# EUROPEAN PATENT SPECIFICATION

(11) **EP 2 648 637 B1**
(45) Date of publication and mention of the grant of the patent: **02.03.2016**
(21) Application number: 11788662.2
(22) Date of filing: 07.11.2011
(51) Int. Cl.: A61B 18/14

(54) **BIPOLAR ELECTROSURGICAL DEVICE**
BIPOLARE ELEKTROCHIRURGISCHE VORRICHTUNG
DISPOSITIF ÉLECTROCHIRURGICAL BIPOLAIRE

(30) Priority: 10.12.2010 US 965495
(43) Date of publication of application: 16.10.2013
(73) Proprietor: Medtronic Advanced Energy LLC, Minneapolis, MN 55432 (US)
(72) Inventor: CONLEY, Brian, M., Portsmouth, NH 03801 (US); GREENLAW, Chad, M., Portsmouth, NH 03801 (US); BERRY, John, W., Portsmoth, NH 03801 (US); ARMY, Joseph, F., Portsmouth, NH 03801 (US); GREELEY, Roger, D., Portsmouth, NH 03801 (US)
(74) Representative: Zimmermann & Partner Patentanwälte mbB
(86) International application number: PCT/US2011/059560
(87) International publication number: WO 2012/078278

(56) References cited:
- US-A1- 2003 171 743
- US-A1- 2004 193 152
- US-A1- 2005 015 085
- US-A1- 2005 267 467
- US-A1- 2005 283 148

## Description

### BACKGROUND OF THE INVENTION

### Field of the Invention

The present invention relates to medical devices, and in particular to an electrosurgical device having a bipolar electrode tip.

### Background Art

Electrosurgical devices use electrical energy, often radio frequency (RF) energy, to cut tissue or to cauterize blood vessels (such procedures are commonly known as "electrocautery"). An electrosurgical device typically has a handle, a shaft extending from the handle having a distal end, and an electrode tip extending from the distal end of the shaft. For example, an electrosurgical device can include an RF ablation needle provided with one or more electrodes for RF ablation of targeted tissue.

Electrosurgical devices can be monopolar or bipolar. In a monopolar device, the device includes one electrode, and a ground pad electrode is located on the patient. Energy applied through the electrode travels through the patient to ground, typically the ground pad. With a bipolar device, the ground pad electrode located on the patient is eliminated and replaced with a second electrode pole as part of the device. These active and return electrodes of a bipolar device are typically positioned close together to ensure that, upon application of electrical energy, current flows directly from the active to the return electrode. An exemplary bipolar device may include laterally-spaced parallel arms extending from a shaft, with one arm including an active electrode and the other arm including a return electrode. The respective electrode or electrodes of such a monopolar or bipolar device may be cone-shaped to allow blunt dissecting of the tissue with the cone tip while also coagulating the tissue with the electrode.

Another exemplary monopolar device is a "sealing hook" device that allows dissection and cauterization. This monopolar device can have an electrode provided on the distal end of the shaft, in which the electrode tip has a blunt spherical side laterally opposite a blade or "hook". The hook may be oriented 90 degrees relative to the shaft. Thus, the hook portion of the electrode can be used for dissection, and the blunt sphere side of the electrode can be used for sealing of the tissue.

Bipolar electrosurgical devices can be advantageous compared to monopolar devices because the return current path only minimally flows through the patient. In bipolar electrosurgical devices, both the active and return electrode are typically exposed so they may both contact tissue, thereby providing a return current path from the active to the return electrode through the tissue. Also, the depth of tissue penetration may be advantageously less with a bipolar device than with a monopolar device. On the other hand, a disadvantage of the bipolar device is that the two electrodes on the device increase the size of the device, such that the device may not be able to be used for certain procedures, such as, for example, laparoscopic surgery.

US 2003/0171743 A1 describes systems and method for electrosurgically promoting blood flow to tissue. US 2005/0267467 A1 describes a conforming electrode catheter and methods for ablation. US 2004/0193152 A1 describes a windowed thermal ablation probe.

### BRIEF SUMMARY OF THE INVENTION

The present invention provides a bipolar electrosurgical device. In one embodiment, the device includes a shaft having a proximal end and a distal end, and an electrode tip coupled to the distal end of the shaft, wherein at least a portion of the electrode tip extends distally beyond the distal end of the shaft and includes a substantially conically-shaped portion. The portion of the electrode tip that extends beyond the distal end of the shaft includes a first electrode, a second electrode, and an insulator disposed between the first electrode and the second electrode. The first electrode is configured to be an active electrode and the second electrode is configured to be a return electrode, and the substantially conically-shaped portion includes at least a portion of each of the first electrode and the second electrode.

In another embodiment, the bipolar electrosurgical device includes a shaft having a proximal end and a distal end, and an electrode tip coupled to the distal end of the shaft, wherein at least a portion of the electrode tip extends distally beyond the distal end of the shaft and includes a substantially conically-shaped portion. The portion of the electrode tip includes a first electrode, a second electrode, and an insulator disposed between the first electrode and the second electrode. The first electrode is configured to be an active electrode and the second electrode is configured to be a return electrode. The substantially conically-shaped portion includes at least a portion of one of the first electrode and the second electrode, and the distal end of the shaft includes a fluid outlet opening in fluid communication with a fluid source, the fluid outlet opening being configured to provide fluid from the fluid source onto an area proximate the first electrode and the second electrode.

In another embodiment, the bipolar electrosurgical device includes a shaft having a longitudinal axis, a proximal end and a distal end, and an electrode tip including a first electrode, a second electrode, and an insulator, the insulator being disposed between the first electrode and the second electrode. At least a portion of the electrode tip is coupled to and extends distally beyond the distal end of the shaft. The extending portion of the electrode tip includes a spherical portion and a cylindrical portion that protrudes from the spherical portion at a non-zero angle with respect to the longitudinal axis of the shaft. The first electrode is configured to be an active electrode and the second electrode is configured to be a return electrode. In some embodiments, the distal end of the shaft includes a fluid outlet opening in fluid communication with a fluid source, the fluid outlet opening being configured to provide fluid from the fluid source onto an area proximate the first electrode and the second electrode.

The present invention also provides a method of treating tissue using electrical energy in which radio frequency energy is provided to a bipolar electrode tip of an electrosurgical device. The bipolar electrode tip includes an active electrode and a return electrode separated by an insulator, and the targeted tissue is contacted with the energized bipolar electrode tip. The bipolar electrode tip can include a conically-shaped portion including at least a portion of each of the first electrode and the second electrode, or a spherical portion and a cylindrical portion that protrudes from the spherical portion at a non-zero angle with respect to a longitudinal axis of the shaft.

### BRIEF DESCRIPTION OF THE DRAWINGS/FIGURES

The accompanying drawings, which are incorporated herein and form a part of the specification, illustrate the present invention and, together with the description, further serve to explain the principles of the invention and to enable a person skilled in the pertinent art to make and use the invention. In the drawings, like reference numbers, letters, or renderings indicate identical or functionally similar elements.
Figure 1 depicts an exemplary electrosurgical device according to an embodiment of the present invention.
Figure 2 depicts a bipolar tip assembly according to an embodiment of the present invention.
Figure 3 depicts an exploded view of the tip assembly depicted in Figure 2.
Figure 4 depicts a top view of the tip assembly depicted in Figure 2.
Figure 5 depicts a side view of the tip assembly depicted in Figure 2.
Figure 6 depicts a cross-sectional view of a bipolar tip assembly according to an embodiment of the present invention.
Figure 7 depicts a front view of the tip assembly depicted in Figure 2 along with an enlarged view of a portion of the front view.
Figure 8 depicts a bipolar tip assembly according to an embodiment of the present invention.
Figure 9 depicts a bipolar tip assembly according to an embodiment of the present invention.
Figure 10 depicts a bipolar tip assembly according to an embodiment of the present invention.
Figure 11 depicts an exemplary electrosurgical device according to an embodiment of the present invention.
Figure 12 depicts a bipolar tip assembly of the device of Figure 11, according to an embodiment of the present invention.
Figure 13 depicts an exploded view of the tip assembly depicted in Figure 12.
Figure 14 depicts a bipolar tip assembly according to an embodiment of the present invention.
Figure 15 depicts a bipolar tip assembly according to an embodiment of the present invention.
Figure 16 depicts a bipolar tip assembly according to an embodiment of the present invention.

### DETAILED DESCRIPTION OF THE INVENTION

Unless defined otherwise, all technical and scientific terms used herein have the same meaning as commonly understood by one of ordinary skill in the art to which this invention belongs. In case of conflict, the present application including the definitions will control. Also, unless otherwise required by context, singular terms shall include pluralities and plural terms shall include the singular.

The term "invention" or "present invention" as used herein is a non-limiting term and is not intended to refer to any single embodiment of the particular invention but encompasses all possible embodiments as described in the application.

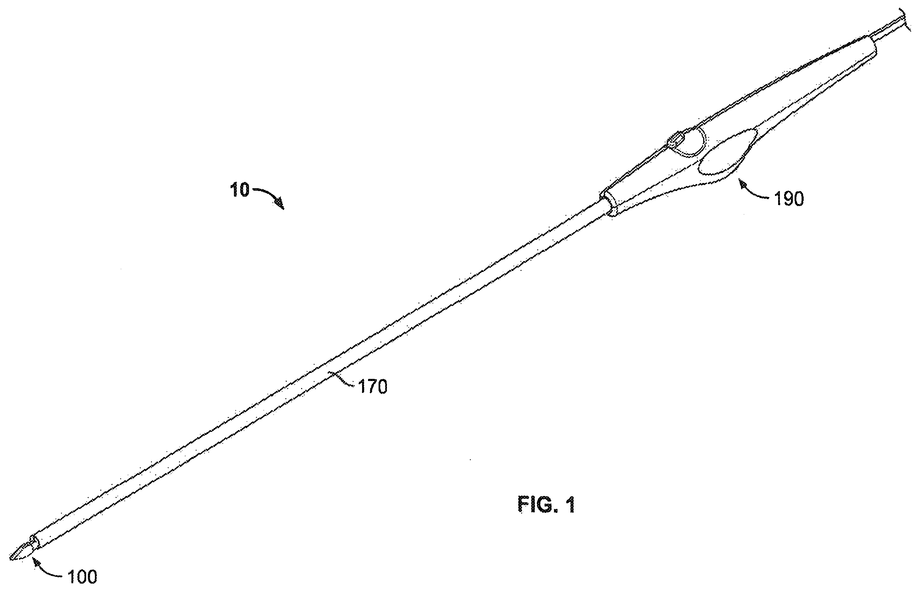

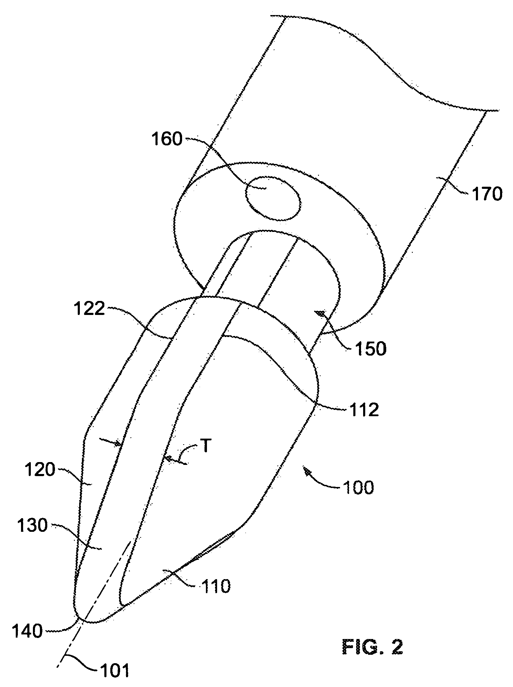

Figure 1 depicts an exemplary electrosurgical device 10 according to the present invention. Device 10 includes a handle 190, a shaft 170, and a bipolar tip assembly 100. Figure 2 depicts bipolar tip assembly 100 in detail. Bipolar tip assembly 100 includes a first electrode 110, a second electrode 120, and an insulator 130. Insulator 130 is positioned between first electrode 110 and second electrode 120. A particular benefit of bipolar tip assemblies of the present invention is that they incorporate bipolar electrodes in a single tip assembly, as will be further outlined in the discussion of the embodiments that follow. Such incorporation of a bipolar assembly of the present invention in place of prior known bipolar electrodes can leave a smaller footprint on treated tissue, can be more conducive for laparoscopic applications, and can facilitate more precise dissections. While Figure 1 depicts an exemplary electrosurgical device that can be used with bipolar tip assembly 100, it should be understood that bipolar tip assembly 100, and the other embodiments of tip assemblies described herein, can be used in conjunction with other bipolar electrosurgical devices known in the art. For example, devices disclosed in U.S. Patent Application Publication No. 2005/0015085 A1 can be modified to incorporate the bipolar tip assemblies disclosed herein.

Each of first electrode 110 and second electrode 120 is configured to be connected to a power source, for example a radio-frequency (RF) power generator. Bipolar tip assembly 100 is bipolar, meaning that first electrode 110 and second electrode 120 can be operatively connected to the power source such that one of the first electrode 110 and the second electrode 120 is an active electrode, while the other of the first electrode 110 and the second electrode 120 is a return electrode. Such connection can be established at proximal ends of first electrode 110 and second electrode 120, for example. Hardware associated with such connection may be contained within shaft 170, for example. In use, RF energy applied to bipolar tip assembly 100 will travel between the active electrode and the return electrode due to a voltage gradient created therebetween, thus traveling over insulator 130. Insulator 130 may be of varying thickness; however, preferably, a thickness T of insulator 130 is substantially uniform. A substantially uniform thickness T provides a substantially uniform distance between first electrode 110 and second electrode 120, thereby contributing to a substantially uniform voltage gradient therebetween, which may be desirable for particular applications of tip assembly 100.

For example, when applied to an electrically conductive surface, such as human tissue during a tissue treatment procedure, laparoscopic procedures, or solid organ resections, electric current will travel, for example, from first electrode 110 (acting as the active electrode) through the tissue, to second electrode 120 (acting as the return electrode). The travel of electrical energy through the tissue heats the tissue via electrical resistance heating. The natural electrical resistance of the tissue causes applied RF energy to be absorbed and transformed into thermal energy via accelerated movement of ions as a function of the tissue's electrical resistance. The level of electrical power used in conjunction with bipolar tip assembly 100 can be varied and optimized for a particular application, and, if sufficiently high, can generate heat sufficient to dissect, coagulate, or otherwise heat-treat the tissue to which it is applied, which can render the tissue suitable for a variety of surgical procedures, such as, for example, blunt dissection. Including first electrode 110 and second electrode 120 together on a single bipolar tip assembly 100 allows for electrical treatment of tissue as described, and concomitant blunt dissection of the treated tissue with bipolar tip assembly 100. Exemplary tissue treatment procedures that can employ the bipolar electrosurgical devices of the present invention include, for example, dissection and coagulation as mentioned above, as well as blunt dissection with coagulation, spot coagulation, and coagulation of large tissue planes.

In order to prevent undesirable thermal damage to tissue (such as, for example, desiccation and char formation, which can occur at temperatures in excess of 100°C, particularly, if there is no fluid present at the tissue being treated), it may be desired to maintain consistent temperature at the tissue being treated. Because electrical resistance of tissue can change as the tissue is dissected, coagulated, or otherwise heat-treated, in order to maintain consistent temperature of the tissue various parameters may have to be adjusted. For example, voltage applied to the electrodes can be varied. In a preferred embodiment, an electrically conductive fluid is applied. The electrically conductive fluid can act as a heat sink, absorbing and carrying away excess or undesirable thermal energy. The electrically conductive fluid can also provide electrical dispersion by distributing the applied current over a larger surface area, thereby limiting the potential for undesirable thermal concentration. Moreover, the electrically conductive fluid can be used to help maintain temperatures within ranges conducive to coagulation of tissue (e.g., temperatures hot enough to denature the collagen and most soft tissue and bone, however not so hot that tissue is damaged to such an extent that it cannot be easily absorbed back into the body during a healing process) as opposed to charred, desiccated tissue. Collagen shrinkage, which causes coagulation, is a function of time and temperature. At 100°C, coagulation occurs substantially instantaneously, and at higher temperatures, there will also be coagulation. However, coagulation can begin at lower temperatures than 100°C, but the coagulation may occur more gradually. Without fluid (e.g., saline) present at the tissue being treated, temperatures can quickly rise above 100°C, and at such higher temperatures there is a greater likelihood of tissue sticking and charring. As one of skill in the art would appreciate, the time and temperature applied can be varied to suit a particular use.

Under some circumstances, the temperature deep in tissue can rise quickly past the 100°C coagulation point even though the electrode/tissue interface is at 100°C (as it may be maintained by application of a saline flow with a boiling point of approximately 100°C). This manifests itself as "popping," as steam generated deep in the tissue boils too fast and erupts toward the surface. To effectively treat thick tissues, it can be advantageous to have the ability to pulse the RF power on and off. In some embodiments, a switch can be provided on the control device or custom generator to allow the user to select a "pulse" mode of the RF power whereby the RF power to the electrosurgical device is repeatedly turned on and off. Moreover, in some embodiments, the control device or custom generator can supply pulsed RF power. As known in the art, the RF power system can be controlled by suitable software to obtain desired power delivery characteristics.

In some embodiments, to further affect the temperature of target tissue, it may be desirable to control the temperature of the conductive fluid before it is released from the electrosurgical device. In some embodiments, a heat exchanger is provided for outgoing saline flow to either heat or chill the saline. Pre-heating the saline to a predetermined level below boiling reduces the transient warm-up time of the device as RF energy is initially turned on, thereby reducing the time to cause coagulation of tissue. Alternatively, pre-chilling the saline is useful when the surgeon desires to protect certain tissues at the electrode/tissue interface and to treat only deeper tissue. One exemplary application of this embodiment is the treatment of varicose veins, where it is desirable to avoid thermal damage to the surface of the skin. At the same time, treatment is provided to shrink underlying blood vessels using thermal coagulation. The temperature of the conductive fluid prior to release from the surgical device can therefore be controlled, to provide the desired treatment effect.

In order to take advantage of these and other beneficial effects of an electrically conductive fluid, the electrically conductive fluid should be applied in close proximity to bipolar tip assembly 100, and should create a fluid (and consequently electrical) connection between first electrode 110 and second electrode 120. This ensures that electrical energy is conducted through the electrically conductive fluid, and associated thermal energy is applied to the tissue. In some embodiments of the present invention, a fluid outlet opening 160 is provided in shaft 170, in close proximity to bipolar tip assembly 100. Fluid outlet opening 160 can be in fluid communication with a fluid source, such as a fluid-filled bladder, reservoir, or pump. Fluid outlet opening 160 may include a single orifice, or may include multiple orifices. In some embodiments, fluid outlet opening 160 is provided in insulator 130, between first electrode 110 and second electrode 120. Fluid outlet opening 160 may also include a permeable material to weep the fluid, thereby helping control the flow and application of the fluid. The flow rate of the electrically conductive fluid can affect the thermal characteristics of the tissue. For example, an uncontrolled or abundant flow rate can provide too much electrical dispersion and cooling at the electrode/tissue interface. On the other hand, a flow rate that is too low could lead to excessive heat and arcing. Suitable techniques for controlling the flow rate of the electrically conductive fluid as desired can be applied to the present invention, and such techniques would be recognized by one of skill in the art.

In a preferred embodiment, saline is used as the electrically conductive fluid, however other electrically conductive fluids may be used alternatively or additionally, consistent with the present invention. While a conductive fluid is preferred, as will become more apparent with further reading of this specification, the fluid from fluid outlet opening 160 may also comprise an electrically non-conductive fluid. The use of a non-conductive fluid still provides certain advantages over the use of a dry electrode including, for example, reduced occurrence of tissue sticking to the electrodes of the tip assemblies disclosed herein, and cooling of the electrodes and/or tissue. Therefore, it is also within the scope of the invention to include the use of a non-conducting fluid, such as, for example, deionized water and lactated ringers.

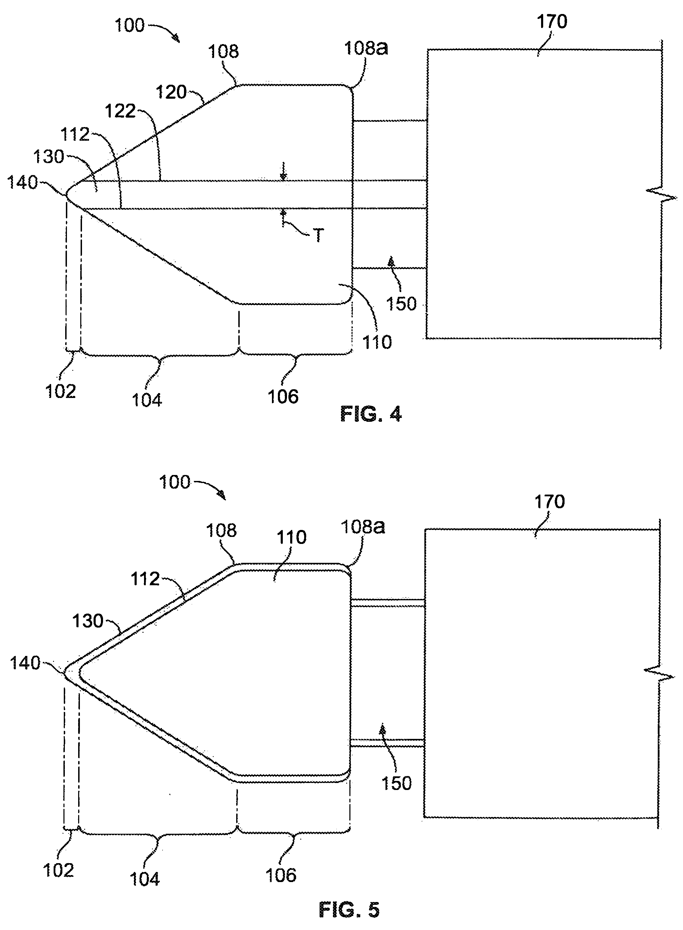

In some embodiments, a distal end of bipolar tip assembly 100 is substantially in the shape of a cone (see Figures 1-8). As shown in Figures 2-6, and 8-10, and in particular Figures 4 and 5, cone-shaped bipolar tip assembly 100 preferably includes a spherical portion 102 at a tip end portion 140, which provides a smooth, blunt contour outer surface. More specifically, as shown, spherical portion 102 provides a domed hemispherical surface portion.

The surface of spherical portion 102 connects tangentially to a surface of a substantially conical portion 104, which is disposed proximally with respect to spherical portion 102. Conical portion 104 is of a concentric cone shape, and can be conical or frustoconical, with spherical portion 102 providing a blunt apex at a distal end of conical portion 104. In some embodiments, however, spherical portion 102 is not included, thereby providing bipolar tip assembly 100 with a tip end portion 140 that has a pointed surface defined by a distal end of conical portion 104, which can be particularly advantageous for tissue dissection procedures. In an embodiment where conical portion 104 is frustoconical, spherical portion 102 can also be omitted, thereby providing bipolar tip assembly 100 with a tip end portion 140 having a flat surface of circular or oval cross-section defined by a distal end of the frustum of conical portion 104. At a proximal end of conical portion 104 the surface of conical portion 104 could connect tangentially, via a radius 108, to a surface of a cylindrical portion 106, which is disposed proximally with respect to conical portion 104. A proximal end of conical portion 106 may include a radius 108a, to avoid defining a sharp edge.

In the embodiment of Figures 2-5, for example, insulator 130 of cone-shaped bipolar tip assembly 100 extends longitudinally along substantially the center of cone-shaped bipolar tip assembly 100, while first electrode 110 and second electrode 120 are positioned on respective laterally opposite sides of insulator 130 and form the balance of the cone shape. In this configuration, tip end portion 140 of cone-shaped bipolar tip assembly 100 is formed by insulator 130. This configuration can be functionally beneficial when cone-shaped bipolar tip assembly 100 is used for a tissue treatment procedure because tip end portion 140 may be the first portion of cone-shaped bipolar tip assembly 100 to come into contact with tissue. Forming tip end portion 140 with insulator 130 promotes travel of electrical current across insulator 130 at tip end portion 140, thereby promoting application of electrical energy to the tissue at first contact. It should be noted, however, that though it can be beneficial to form tip end portion 140 with insulator 130 for at least the reasons outlined above, such a configuration is not necessary, as will be made clear with reference to further embodiments below.

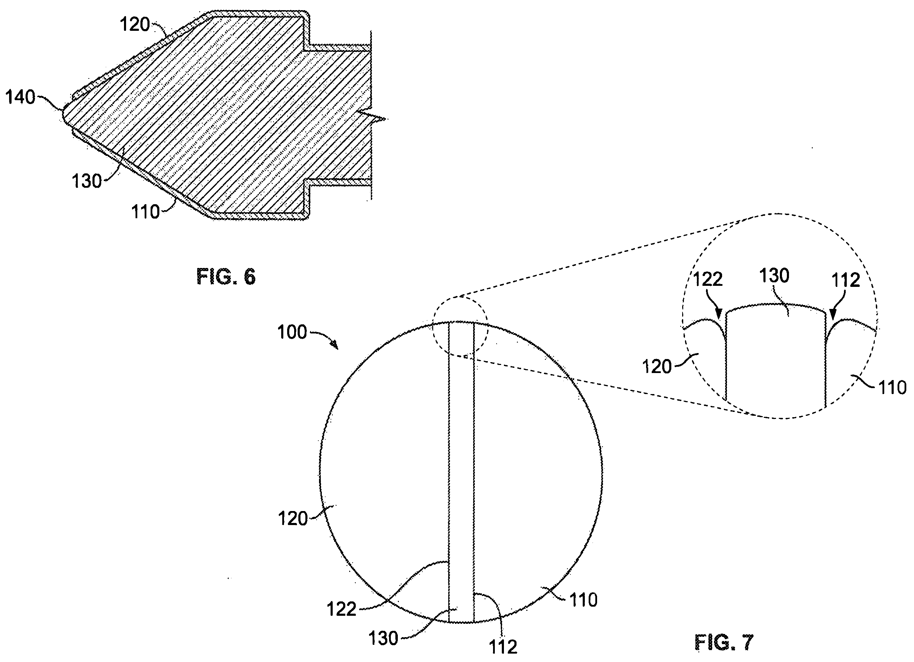

As shown in the side view of Figure 5, insulator 130 is proud of edges of first electrode 110 (see electrode/insulator interface 112), and is also proud of second electrode 120 (not shown in this view). In some embodiments, only a portion of insulator 130 is proud of the edge of first electrode 110 and/or the edge of second electrode 120. In some further embodiments, all or a portion of the exterior surface of insulator 130 is flush with the edge of first electrode 110 and/or the edge of second electrode 120. In some further embodiments, all or a portion of the exterior surface of insulator 130 is recessed at the edge of first electrode 110 at electrode/insulator interface 112 and/or the edge of second electrode 120 at electrode/insulator interface 122 (see Figure 7). Thus, in some embodiments, the exterior surface of insulator 130 can be any one of, or a combination of, proud, flush, and recessed with respect to the edge of first electrode 110, while the exterior surface of insulator 130 can be any one of, or a combination of, proud, flush, and recessed with respect to second electrode 120.

Cone-shaped bipolar tip assembly 100 can be formed of various sizes to suit particular applications as would be apparent to one of skill in the art. For example, cone-shaped bipolar tip assembly 100 can have a maximum diameter of approximately 0.2 inches (approximately 5mm) or approximately 0.4 inches (approximately 10mm), in order to be suitable for use with a similarly-sized trocar. Alternatively, cone-shaped bipolar tip assembly 100 can be formed having other maximum diameters, to suit particular applications.

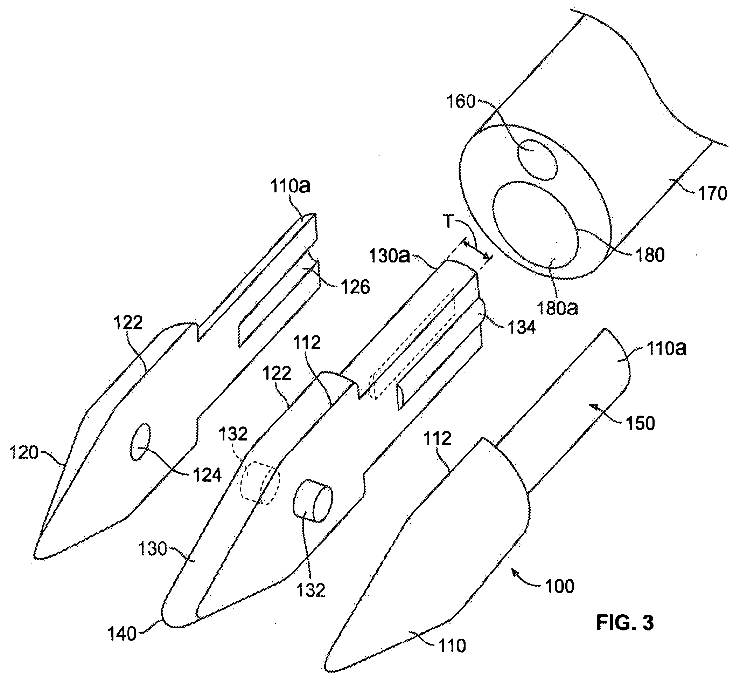

In some embodiments, first electrode 110 and second electrode 120 are solid structural and discrete portions of cone-shaped bipolar tip assembly 100, as shown in the exploded view of Figure 3. First electrode 110 and second electrode 120 may be formed of any suitable material, for example, a biocompatible conductor such as stainless steel or titanium. In other embodiments, first electrode 110 and second electrode 120 are formed of a conductive ink applied to the surface of a substrate. In some embodiments, the substrate may be the same material that forms insulator 130. In such an embodiment, the entire cone-shaped bipolar tip assembly 100 may be a monolithic structure with the material of insulator 130 substantially forming the entire cone-shaped bipolar tip assembly 100. Conductive ink is applied to cover distinct portions of the insulator material, thereby forming first electrode 110 and second electrode 120 on cone-shaped bipolar tip assembly 100 (see Figure 6, which depicts a cross-sectional view of a cone-shaped bipolar tip assembly 100 where first electrode 110 and second electrode 120 are formed of conductive ink). The use of conductive ink can be beneficial in manufacturing smaller bipolar tip assemblies 100, as fewer discrete structural components may be required to be manufactured and assembled. The conductive ink can include ink or paint formed of conductive materials such as, for example, powdered or flaked silver, carbon, or similar materials.

In some embodiments, one of first electrode 110 and second electrode 120 is a solid structural portion of cone-shaped bipolar tip assembly 100, while the other of first electrode 110 and second electrode 120 is formed of a conductive ink or paint applied to the surface of insulator 130.

In some embodiments, insulator 130 is a solid structural portion of cone-shaped bipolar tip assembly 100, as shown in the exploded view of Figure 3. Insulator 130 may be formed of any suitable material. Preferably, insulator 130 is formed of an RF-resistant material (i.e., a material with a high dielectric strength with reference to RF energy), for example, ceramic or TEFLON®. Insulator 130 should be of a suitable thickness to prevent electrical shorts or undesirably high temperatures between first electrode 110 and second electrode 120, such as, for example, from at least about 0.02 inches to at least about 0.03 inches.

First electrode 110 and second electrode 120 can couple to insulator 130 by any suitable technique, including, for example, adhesively or mechanically. In the embodiment shown, insulator 130 includes connection features in the form of protrusions 132 and 134 that interface with respective cavities 124 and 126 of second electrode 120, and with similar cavities (not shown) of first electrode 110. These protrusions 132 and 134 and respective cavities 124 and 126 may interlock, e.g., by press fit, so that first electrode 110, second electrode 120, and insulator 130 are secured together. In some embodiments, these connection features may simply help maintain proper alignment of first electrode 110, second electrode 120, and insulator 130, while other coupling mechanisms (e.g., adhesive or mechanical mechanisms) are used to secure electrode 110, second electrode 120, and insulator 130 together. For example, in some embodiments, first electrode 110 and second electrode 120 are coupled to insulator 130 solely by virtue of the press-fit interface with an electrode-receiving channel of shaft 170. In some embodiments, assembly 100 of first electrode 110, second electrode 120, and insulator 130 is produced by a plastic overmolding process. For example, first electrode 110 and second electrode 120 are held in place in a mold, and a plastic insulative material is injected to form insulator 130 which adheres to electrode 110 and second electrode 120 during the molding process. Assembly 100 is then removed from the mold. The plastic insulative material may be a plastic with an affinity to the metal to promote adhesion thereto.

In particular, shaft 170 can include an electrode-receiving channel 180 having an opening 180a at least at a distal end thereof, for accommodating respective proximal ends 110a, 120a, and 130a of first electrode 110, second electrode 120, and insulator 130. When assembled, respective proximal ends 110a, 120a, and 130a of first electrode 110, second electrode 120, and insulator 130 together form a cylindrically-shaped neck 150 of cone-shaped bipolar tip assembly 100. Opening 180a of electrode-receiving channel 180 can be circular and have a slightly larger or smaller diameter as that of cylindrically-shaped neck 150, such that neck 150 can be accommodated within channel 180 and preferably form a press-fit interface. A press-fit interface between the proximal end of cone-shaped bipolar tip assembly 100 will help secure together shaft 170 and cone-shaped bipolar tip assembly 100, and will interlock and/or help maintain proper alignment of first electrode 110, second electrode 120, and insulator 130. In some embodiments (not shown), neck 150 can be shapes other than cylindrical, and opening 180a of channel 180 can be other shapes other than circular, while still permitting a press-fit interface, if such is intended, as would be appreciated by one of skill in the art. For example, in some embodiments (not shown), neck 150 and opening 180a can have corresponding cross-sections of non-circular shapes (e.g., square or triangular), which can have similar dimensions so as to permit a press-fit interface. A benefit of corresponding cross-sections of non-circular shape is that the corresponding shapes can be keyed to one another so as to limit the potential orientations at which neck 150 will fit into opening 180a, thereby simplifying assembly. In some embodiments, neck 150 and opening 180a can have different cross-sections (e.g., square and circular, respectively), which are dimensioned to still permit a press-fit interface. In some embodiments, adhesives and/or other mechanical attachment mechanisms (e.g., a bayonet locking device) can be used between neck 150 and channel 180 in lieu of or in addition to a press-fit interface.

In some embodiments, shaft 170, from which bipolar tip assembly 100 extends, may be a rigid shaft, a malleable shaft, or an articulating shaft, or any combination thereof such that different portions of the shaft can be any one of rigid, malleable, and articulating. These and other characteristics (e.g., the cross-section geometry) of shaft 170 can be varied as desired or to suit a particular application.

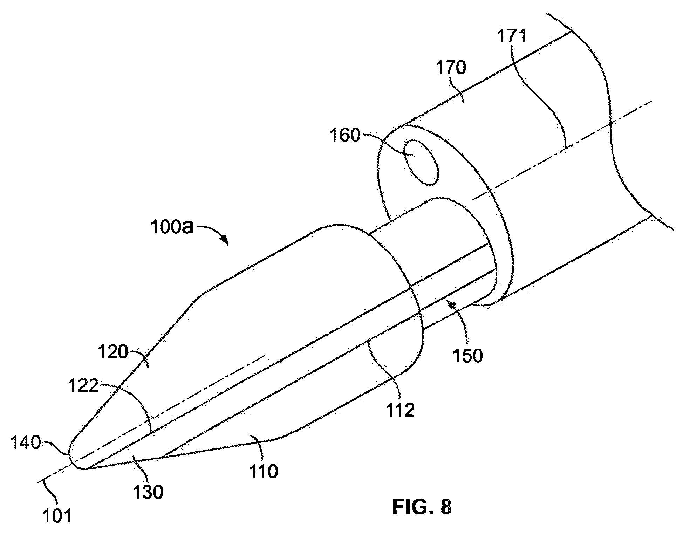

Figure 4 depicts a top view of cone-shaped bipolar tip assembly 100. Figure 5 depicts a side view of cone-shaped bipolar tip assembly 100. As can be appreciated with reference to Figures 4 and 5, in some embodiments tip end portion 140 of cone-shaped bipolar tip assembly 100 is rounded. Details of geometries of some embodiments of cone-shaped bipolar tip assembly 100 have been described above. A rounded tip promotes consistent energy flow between first electrode 110 and second electrode 120, and diminishes the possibility of undesirably concentrating energy at a sharp edge or point. Such concentration of energy may be undesirable as potentially creating a "hot spot" of electrical activity relative to the balance of cone-shaped bipolar tip assembly 100, and increasing the possibility of electrical short between first electrode 110 and second electrode 120. These undesirable effects (and the potential for mitigating them by use of a rounded tip) are particularly applicable in embodiments where the electrode (rather than the insulator) forms the tip end portion 140 (as in, for example, the embodiment of Figure 8, discussed below).

Figure 7 depicts a front view of cone-shaped bipolar tip assembly 100, along with an enlarged view of a portion of the front view (shaft 170 is not shown). As can be appreciated with reference to Figure 7, in some embodiments first electrode 110 and second electrode 120 include rounded (i.e., radiused) edges at a first electrode/insulator interface 112 and a second electrode/insulator interface 122, respectively. As explained above, undesirable concentration of energy can occur at sharp edges. The inclusion of rounded edges on first electrode 110 and second electrode 120 mitigates the potential for such undesirable concentration by minimizing the sharpness that could otherwise exist at this edge. The rounded edges are particularly beneficial at the respective interfaces of first electrode 110 and second electrode 120 with insulator 130, because the interfaces are the areas at which the electrodes are closest together, and consequently the areas at which electrical energy may be concentrated.

In some alternative embodiments, first electrode 110 and second electrode 120 include sharp edges. In some alternative embodiments, a portion of edges of first electrode 110 and second electrode 120 are rounded and a portion of edges of first electrode 110 and second electrode 120 are sharp.

Figures 8-10 depict various other configurations of bipolar tip assembly 100 according to some embodiments presented herein. Such other configurations may be beneficial to a user by providing an electrical field at different areas of bipolar tip assembly 100, which may be advantageous depending on the application and procedure.

In particular, Figure 8 depicts a bipolar tip assembly 100a in which insulator 130 extends longitudinally between first electrode 110 and second electrode 120, similar to the embodiment of Figure 2. However, in the embodiment of Figure 8, insulator 130 is laterally offset from a longitudinal axis 101 of bipolar tip assembly 100, such that tip end portion 140 of bipolar tip assembly 100 is formed by second electrode 120, rather than by insulator 130. Consequently, first electrode 110 has a smaller surface area than second electrode 120.

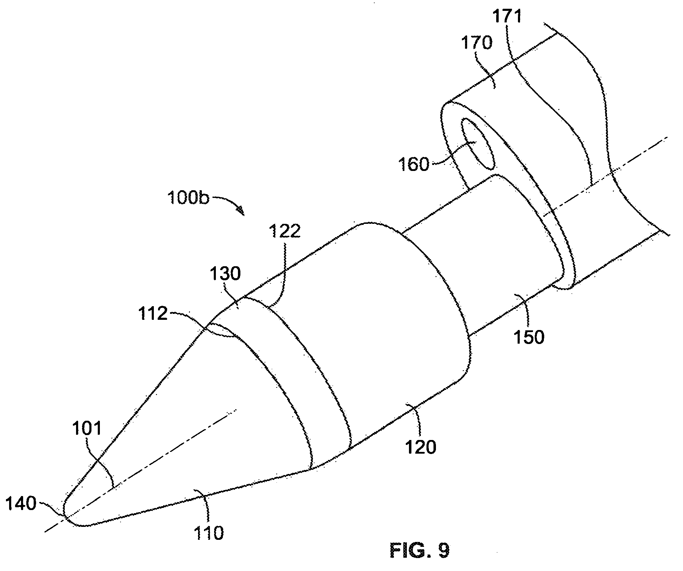

Figure 9 depicts a bipolar tip assembly 100b in which insulator 130 extends transversely with first electrode 110 and second electrode 120 on longitudinally opposite sides of insulator 130. As such, first electrode 110 and second electrode 120 are separated from each other longitudinally. In the embodiment shown, insulator 130 extends substantially perpendicularly with respect to longitudinal axis 101 of the bipolar tip assembly 101b, and likewise extends substantially perpendicularly with respect to a longitudinal axis 171 of shaft 170.

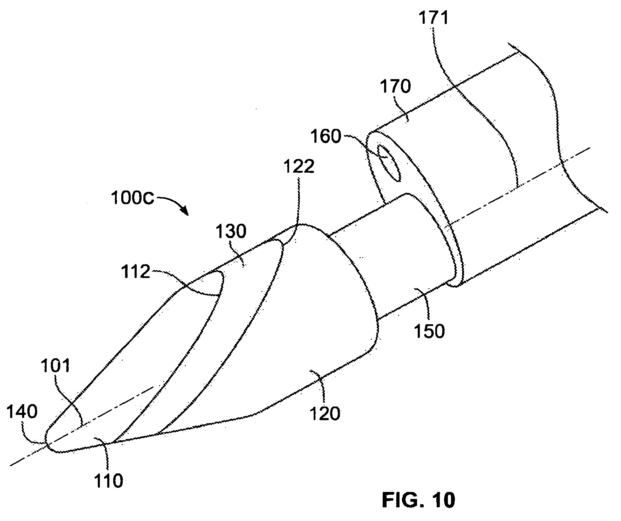

Alternatively, in some embodiments, insulator 130 can extend between first electrode 110 and second electrode 120 at an oblique angle with respect to longitudinal axis 101 and longitudinal axis 171 of shaft 170, as provided in bipolar tip assembly 100c illustrated in Figure 10. In the embodiments of Figures 9 and 10, it should be apparent to one of skill in the art that second electrode 120 and insulator 130 may include an insulated channel along their interiors such that first electrode 110 may be in electrical communication with a power source through the channel.

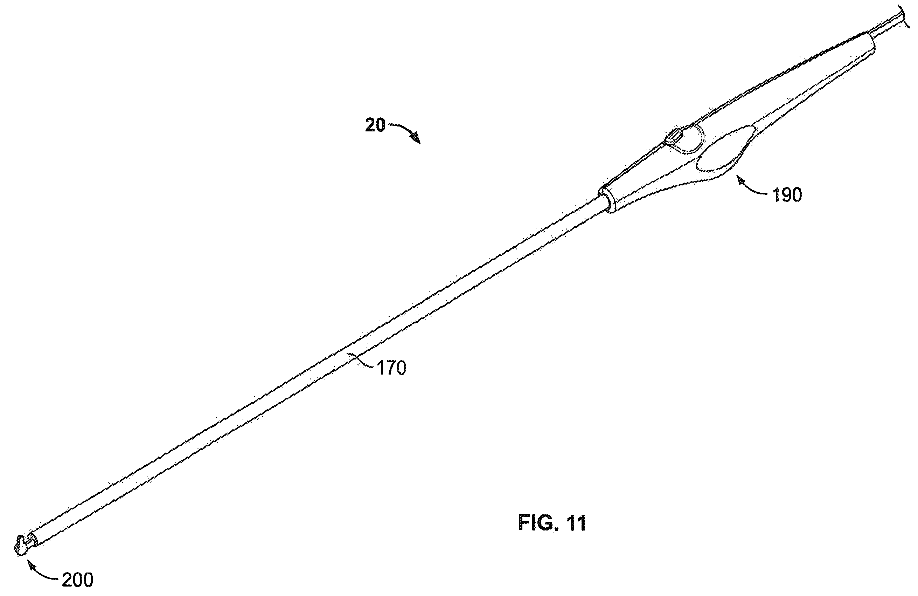

Figures 11-16 depict an exemplary device and tip assemblies according to other embodiments of the present invention. In these figures, elements with similar or identical function and configuration as those previously described are denoted with identical reference numbers, and detailed explanation of such elements may be omitted or abbreviated in the description that follows.

Figure 11 depicts an exemplary electrosurgical device 20 that incorporates a bipolar tip assembly 200 according to an embodiment of the present invention. Device 20 includes a handle 190, a shaft 170, and bipolar tip assembly 200. Device 20 of Figure 11 is similar to device 10 of Figure 1, but is provided with bipolar tip assembly 200. Bipolar tip assemblies 100 and 100a-c and bipolar tip assembly 200 can each be used with device 10 or 20, or other bipolar electrosurgical devices configured to provide an electrical field (e.g., an RF electric field) across active and return electrodes as known in the art. In some embodiments bipolar tip assemblies 100 and 100a-c and bipolar tip assemblies 200 and 200a-c can be detachably coupled to device 10 or 20, and selectively interchanged on device 10 or 20 with each other or with other bipolar tip assemblies, allowing the same device 10 or 20 to be modified for different procedures by changing the tip assembly of the device.

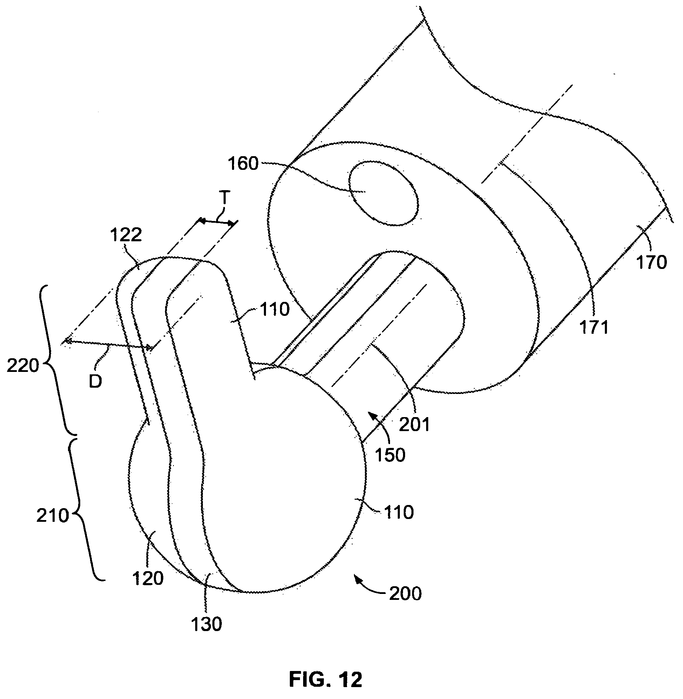

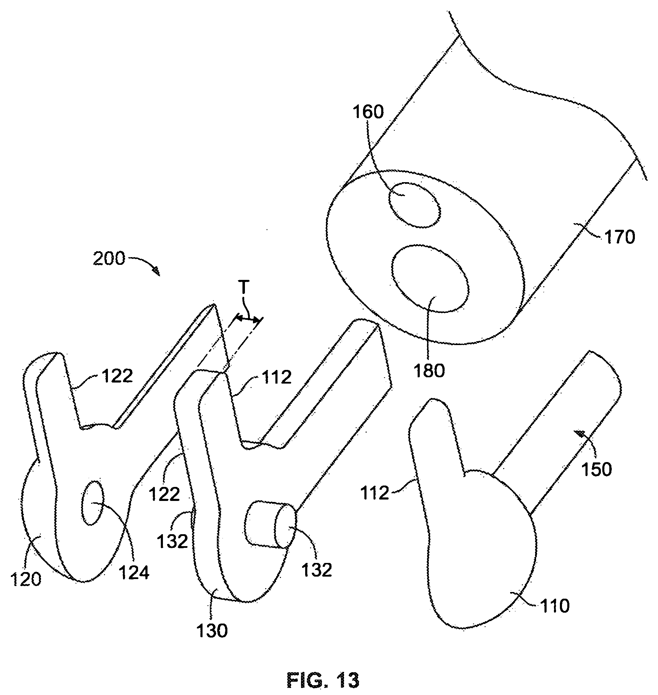

Figure 12 depicts bipolar tip assembly 200 in detail. Figure 13 depicts an exploded view of the embodiment of bipolar tip assembly 200 shown in Figure 12. Bipolar tip assembly 200 includes a spherical portion 210 and a cylindrical portion 220, along with neck portion 150. As shown most clearly in Figure 13, each of first electrode 110, second electrode 120, and insulator 130 can independently and monolithically form a portion of neck 150, spherical portion 210, and cylindrical portion 220. In some embodiments, cylindrical portion 220 protrudes from spherical portion 210 at a non-zero angle with respect to longitudinal axis 171 of the shaft. In some embodiments, cylindrical portion 220 protrudes substantially perpendicularly to longitudinal axis 171 of shaft 170, to create a hook-like configuration. Relative to spherical portion 210, cylindrical portion 220 may have a smaller diameter. In some embodiments, the diameter of cylindrical portion 220 is approximately one-quarter the diameter of spherical portion 210. As would be appreciated by one of skill in the art, however, the absolute and relative sizes of spherical portion 210 and cylindrical portion 220 can be varied to suit a particular application or requirement. Moreover, cylindrical portion 220 can have a circular, elliptical, parabolic, or hyperbolic cross-section, and in some embodiments, cylindrical portion 220 is not a cylinder, but is, for example, a column having a parallelogram cross-section, or a cone. In some embodiments (not shown), a thickness of cylindrical portion 220 tapers from its center to its longitudinal edge so as to form a cutting edge along a length of cylindrical portion. Thus, the cutting edge extends parallel with a longitudinal axis of the cylindrical portion 220 and at a non-zero angle with respect to longitudinal axis 171 of shaft 170 (thereby providing cylindrical portion 220 with a portion that is triangular in cross-section). Such an edge on cylindrical portion 220 can be beneficial for operations involving cutting tissue. In some embodiments, cylindrical portion 220 is omitted.

In the embodiment of Figure 12, insulator 130 extends longitudinally between first electrode 110 and second electrode 120, and forms a portion of each of neck 150, spherical portion 210, and cylindrical portion 220. Insulator 130 is centered relative to a longitudinal axis 201 of bipolar tip assembly 200 and is oriented to divide the remaining portions of neck 150, spherical portion 210, and cylindrical portion 220 of bipolar tip assembly 200 into substantially equal halves, one half being first electrode 110 and the other half being second electrode 120, as best shown in the exploded view of bipolar tip assembly 200 in Figure 13. In the embodiment shown, a thickness T of insulator 130 is less than a diameter of spherical portion 210 and a diameter D of cylindrical portion 220 so as to allow each of first electrode 110 and second electrode 120 to extend along portions of both spherical portion 210 and cylindrical portion 220 of bipolar tip assembly 200. This configuration permits an electrical field to be provided at spherical portion 210 and cylindrical portion 220 when electrodes 110 and 120 are energized, such as with RF energy.

The embodiment of Figure 11, including cylindrical portion 220 protruding perpendicularly from spherical portion 210, can be beneficial in a variety of procedures. The geometries of these portions provide surfaces conducive to both precise dissection and less precise coagulation, which can be gentler than dissection. For example, a user of bipolar tip assembly 200 may pre-treat a region of tissue with spherical portion 210, applying electrical current to and coagulating the tissue. The spherical geometry is well-suited to such use at least because spherical geometry has fewer sharp edges, allowing for more spread out and consistent energy flow while reducing the potential for coagulant buildup on bipolar tip assembly 200. After pre-treating with spherical portion 210, the user may then dissect the pre-treated region of tissue with the more-precise cylindrical portion 220. Other beneficial applications of having the geometries of both spherical region 210 and cylindrical region 220 in a single bipolar tip assembly 200 will be apparent to one of skill in the art.

Bipolar tip assembly 200 can be used in conjunction with a fluid, which, in some embodiments, can be a conductive fluid, as described above with reference to bipolar tip assembly 100, and elements of bipolar tip assembly 200 can be formed and assembled similarly to elements of bipolar tip assembly 100, as described above. For example, either or both of first electrode 110 and second electrode 120 can be formed of stainless steel, and if formed of stainless steel, then each electrode can form a portion of each of neck 150, spherical portion 210, and cylindrical portion 220, which portions together form a monolithic structure constituting the electrode. Moreover, either or both of first electrode 110 and second electrode 120 can be formed of conductive ink. If the electrode(s) are formed of conductive ink, an insulative material can monolithically form all of neck 150, spherical portion 210, and cylindrical portion 220, and conductive ink can be applied to the insulative material to form one or both of first electrode 110 and second electrode 120.

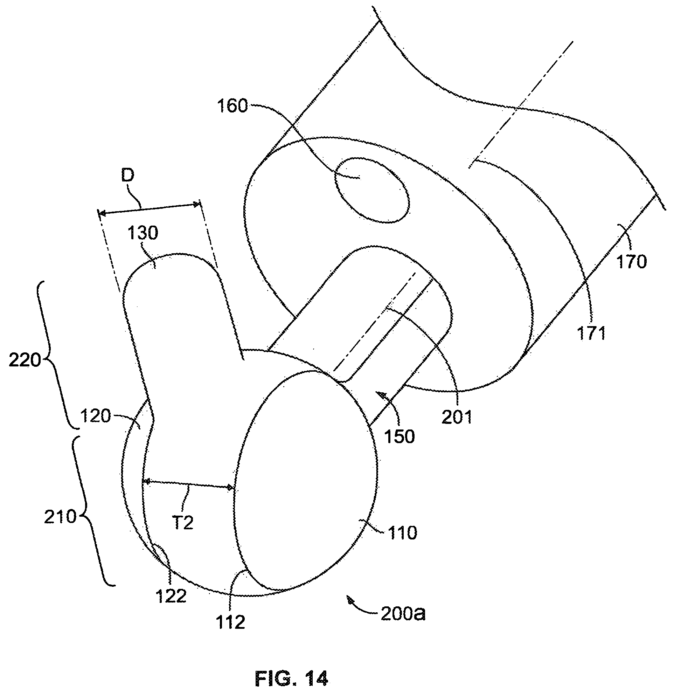

Figures 14-16 depict various other configurations of bipolar tip assembly 200 according to some embodiments presented herein. Such other configurations may be beneficial to a user by providing electrical energy at different areas of bipolar tip assembly 200, allowing bipolar tip assembly 200 to be more easily and effectively used in a variety of different applications and procedures.

Figure 14 depicts a bipolar tip assembly 200a in which insulator 130 extends longitudinally whereby first electrode 110 and second electrode 120 are disposed on laterally opposite sides of insulator 130, similar to bipolar tip assembly 200 of Figure 12. In the embodiment of Figure 14, however, insulator 130 has a thickness T2 that is substantially equal to or greater than diameter D of cylindrical portion 220, such that insulator 130 substantially entirely forms cylindrical portion 220. Such a configuration can be beneficial for particular applications of bipolar tip assembly 200, such as, for example, when cylindrical portion 220 is needed only for pulling or otherwise manipulating tissue without treating it electrically. In such an application, only spherical portion 210 of tip assembly 200a may have the capability of electrically treating tissue.

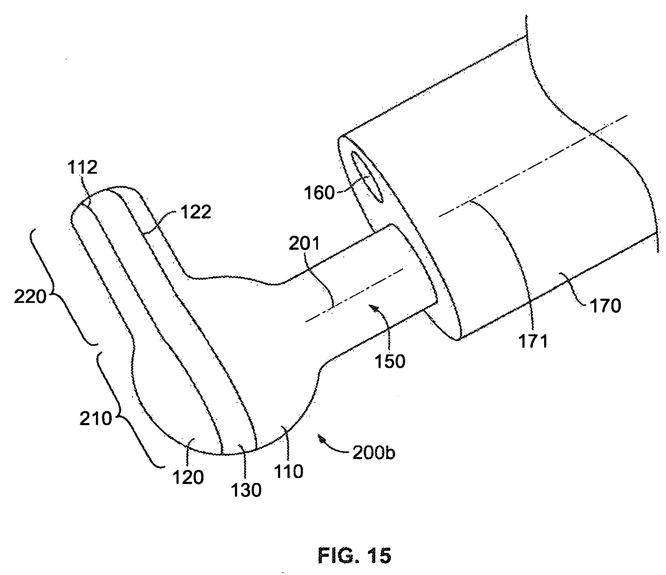

Figure 15 depicts a bipolar tip assembly 200b in which insulator 130 extends transversely whereby first electrode 110 and second electrode 120 are disposed on longitudinally opposite sides of insulator 130. Similar to bipolar tip assembly 100b, first electrode 110 and insulator 130 may include an insulated channel along their interiors such that second electrode may be in electrical communication with a power source through the channel.

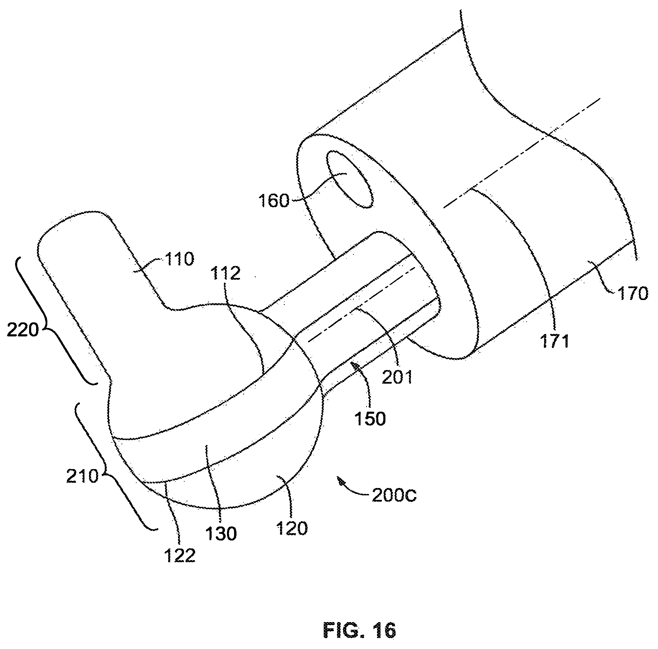

Figure 16 depicts a bipolar tip assembly 200c in which insulator 130 extends longitudinally between first electrode 110 and second electrode 120, similar to bipolar tip assembly 200 of Figure 12. However, in this embodiment, first electrode 110 has a greater surface area than second electrode 120. In particular, insulator 130 of bipolar tip assembly 200c forms a portion of only neck 150 and spherical portion 210, but does not form a portion of cylindrical portion 220. Consequently, insulator 130 is oriented to divide the remaining portions of bipolar tip assembly 200c (including the remaining portions of neck 150 and spherical portion 210 and the entirety of cylindrical portion 220) into two unequal portions that respectively form first electrode 110 and second electrode 120. In the embodiment shown, first electrode 110 constitutes the entirety of cylindrical portion 220 and a portion of each of spherical portion 210 and neck 150, whereas second electrode 120 constitutes a portion of spherical portion 210 and neck 150.

The foregoing description of the specific embodiments of the devices and methods described with reference to the Figures will so fully reveal the general nature of the invention that others can, by applying knowledge within the skill of the art, readily modify and/or adapt for various applications such specific embodiments, without undue experimentation, without departing from the general concept of the present invention. For example, in some embodiments the device 10 or 20 can be used as a selectably monopolar or bipolar device, switchable between a monopolar mode and a bipolar mode. In the monopolar mode, at least one of first electrode 110 and second electrode 120 is connected to a power generator so as to deliver energy as a monopolar (active) electrode, and there is no return electrode on the device (rather, a ground pad on the patient may be used as known in the art). Monopolar devices can be particularly suitable for cutting tissue. For example, in the embodiment of Figure 8, second electrode 120 can serve as a monopolar electrode, and in the embodiments of Figures 9, 10 or 16, first electrode 110 can serve as a monopolar electrode, thereby making these embodiments of devices 10 and 20 particularly useful in tissue cutting procedures. In some embodiments, the monopolar electrode may be supplied with RF energy (including pulsed RF energy), ultrasonic energy, or any other suitable energy for cutting tissue.

Therefore, such adaptations and modifications are intended to be within the meaning and range of equivalents of the disclosed embodiments, based on the teaching and guidance presented herein. It is to be understood that the phraseology or terminology herein is for the purpose of description and not of limitation, such that the terminology or phraseology of the present specification is to be interpreted by the skilled artisan in light of the teachings and guidance. The breadth and scope of the present invention should not be limited by any of the above-described exemplary embodiments, but should be defined only in accordance with the following claims and their equivalents.

## Claims

1. A bipolar electrosurgical device (10) comprising:
a shaft (170) having a proximal end and a distal end; and
an electrode tip (100) coupled to the distal end of the shaft, wherein at least a portion of the electrode tip extends distally beyond the distal end of the shaft,
wherein the portion of the electrode tip (100) comprises a first electrode (110), a second electrode (120), and an insulator (130) disposed between the first electrode and the second electrode,
wherein the first electrode (110) is configured to be an active electrode and the second electrode (120) is configured to be a return electrode; and
wherein at least the portion of the electrode tip includes a substantially conically-shaped portion (104) which includes at least a portion of each of the first electrode and the second electrode, or
the portion of the electrode tip includes a spherical portion (102, 210) and a cylindrical portion (106, 220) that protrudes from the spherical portion (102, 210) at a non-zero angle with respect to a longitudinal axis of the shaft.

2. The device of claim 1, wherein the distal end of the shaft includes a fluid outlet opening (160) in fluid communication with a fluid source, the fluid outlet opening being configured to provide fluid from the fluid source onto an area proximate the first electrode and the second electrode.

3. The device of claim 2, wherein the fluid is electrically conductive and/or includes saline.

4. The device of claim 1, wherein the first electrode and the second electrode have rounded edges at respective interfaces with the insulator.

5. The device of claim 1, wherein the insulator extends parallel to a longitudinal axis of the electrode tip, and wherein the first electrode and the second electrode are disposed on laterally opposite sides of the insulator; wherein optionally the insulator is centered with respect to the longitudinal axis of the electrode tip, and/or the insulator has a uniform thickness.

6. The device of claim 1, wherein the electrode tip (100) comprises a spherical portion (102) at a distal end thereof, wherein a distal end of the substantially conically-shaped portion (104) is coupled to a proximal end of the spherical portion (102), and wherein a distal end of a cylindrical portion (106) is coupled to a proximal end of the substantially conically-shaped portion (104).

7. The device of claim 1, wherein the insulator (140) extends transversely with respect to a longitudinal axis of the electrode tip (100), and wherein the first electrode and the second electrode are disposed on longitudinally opposite sides of the insulator; wherein optionally the insulator extends at an oblique angle with respect to the longitudinal axis of the electrode tip.

8. The device of claim 1, wherein at least one of the first electrode and the second electrode is formed of a conductive ink disposed on a substrate.

9. The device of claim 2, wherein the electrode tip comprises a spherical portion at a distal end thereof, wherein a distal end of the substantially conically-shaped portion is coupled to a proximal end of the spherical portion, and wherein a distal end of a cylindrical portion is coupled to a proximal end of the substantially conically-shaped portion; wherein optionally the substantially conically-shaped portion includes substantially the entirety of the first electrode, and wherein the cylindrical portion includes substantially the entirety of the second electrode.

10. The device of claim 2, wherein the insulator extends parallel to a longitudinal axis of the electrode tip, and wherein the first electrode and the second electrode are disposed on laterally opposite sides of the insulator.

11. The device of claim 2, wherein the fluid is electrically conductive and the first electrode and the second electrode are configured to be energized by radio-frequency energy.

12. The device of claim1, wherein the cylindrical portion protrudes from the spherical portion substantially perpendicularly to the longitudinal axis of the shaft.

13. The device of claim 1, wherein the spherical portion includes at least a portion of each of the first electrode, the second electrode, and the insulator, and wherein the cylindrical portion includes at least a portion of each of the first electrode, the second electrode, and the insulator.

14. The device of claim 1, wherein the first electrode and the second electrode have rounded edges at respective interfaces with the insulator, and wherein the cylindrical portion has a rounded edge at an end portion thereof.

15. The device of claim 7, wherein the insulator forms substantially the entire cylindrical portion, and the spherical portion includes at least a portion of each of the first electrode and the second electrode.

## Patentansprüche

1. Bipolares elektrochirurgisches Gerät (10), umfassend:
einen Schaft (170), der ein proximales Ende und ein distales Ende aufweist; und
eine Elektrodenspitze (100), die mit dem distalen Ende des Schafts gekoppelt ist, wobei mindestens ein Teil der Elektrodenspitze sich distal über das distale Ende des Schafts hinaus erstreckt,
wobei der Teil der Elektrodenspitze (100) eine erste Elektrode (110), eine zweite Elektrode (120) und ein Isolierstück (130), das zwischen der ersten Elektrode und der zweiten Elektrode angeordnet ist, umfasst,
wobei die erste Elektrode (110) so konfiguriert ist, dass sie eine aktive Elektrode ist, und die zweite Elektrode (120) so konfiguriert ist, dass sie eine Gegenelektrode ist; und
wobei mindestens der Teil der Elektrodenspitze einen im Wesentlichen konisch geformten Teil (104) einschließt, der mindestens einen Teil sowohl der ersten Elektrode als auch der zweiten Elektrode einschließt, oder
der Teil der Elektrodenspitze schließt einen kugelförmigen Teil (102, 210) und einen zylindrischen Teil (106, 220), der aus dem kugelförmigen Teil (102, 210) in einem nicht Null betragenden Winkel in Bezug auf eine Längsachse des Schafts herausragt, ein.

2. Gerät nach Anspruch 1, wobei das distale Ende des Schafts eine Flüssigkeitsauslassöffnung (160) einschließt, die in fluider Kommunikation mit einer Flüssigkeitsquelle steht, wobei die Flüssigkeitsauslassöffnung so konfiguriert ist, dass sie Flüssigkeit von der Flüssigkeitsquelle an einem Bereich in der Nähe der ersten Elektrode und der zweiten Elektrode zur Verfügung stellt.

3. Gerät nach Anspruch 2, wobei die Flüssigkeit elektrisch leitfähig ist und/oder Kochsalzlösung enthält.

4. Gerät nach Anspruch 1, wobei die erste Elektrode und die zweite Elektrode an jeweiligen Schnittstellen mit dem Isolierstück abgerundete Kanten aufweisen.

5. Gerät nach Anspruch 1, wobei sich das Isolierstück parallel zu einer Längsachse der Elektrodenspitze erstreckt, und wobei die erste Elektrode und die zweite Elektrode auf lateral gegenüberliegenden Seiten des Isolierstücks angeordnet sind; wobei optional das Isolierstück in Bezug auf die Längsachse der Elektrodenspitze zentriert ist und/oder das Isolierstück eine gleichmäßige Dicke aufweist.

6. Gerät nach Anspruch 1, wobei die Elektrodenspitze (100) einen kugelförmigen Teil (102) an einem distalen Ende davon umfasst, wobei ein distales Ende des im Wesentlichen konisch geformten Teils (104) mit einem proximalen Ende des kugelförmigen Teils (102) gekoppelt ist, und wobei ein distales Ende eines zylindrischen Teils (106) mit einem proximalen Ende des im Wesentlichen konisch geformten Teils (104) gekoppelt ist.

7. Gerät nach Anspruch 1, wobei das Isolierstück (140) sich in Bezug auf eine Längsachse der Elektrodenspitze (100) quer erstreckt, und wobei die erste Elektrode und die zweite Elektrode auf längs gegenüberliegenden Seiten des Isolierstücks angeordnet sind; wobei sich das Isolierstück optional in einem schiefen Winkel in Bezug auf die Längsachse der Elektrodenspitze erstreckt.

8. Gerät nach Anspruch 1, wobei mindestens eine Elektrode der ersten Elektrode und der zweiten Elektrode durch leitfähige Tinte, die auf einem Substrat disponiert ist, gebildet wird.

9. Gerät nach Anspruch 2, wobei die Elektrodenspitze einen kugelförmigen Teil an einem distalen Ende davon umfasst, wobei ein distales Ende des im Wesentlichen konisch geformten Teils mit einem proximalen Ende des kugelförmigen Teils gekoppelt ist, und wobei ein distales Ende des zylindrischen Teils mit einem proximalen Ende des im Wesentlichen konisch geformten Teils gekoppelt ist; wobei optional der im Wesentlichen konisch geformte Teil im Wesentlichen die Gesamtheit der ersten Elektrode einschließt, und wobei der zylindrische Teil im Wesentlichen die Gesamtheit der zweiten Elektrode einschließt.

10. Gerät nach Anspruch 2, wobei das Isolierstück sich parallel zu einer Längsachse der Elektrodenspitze erstreckt, und wobei die erste Elektrode und die zweite Elektrode auf lateral gegenüberliegenden Seiten des Isolierstücks angeordnet sind. 11.

11. Gerät nach Anspruch 2, wobei die Flüssigkeit elektrisch leitfähig ist und die erste Elektrode und die zweite Elektrode so konfiguriert sind, dass sie durch Hochfrequenzenergie mit Energie versorgt werden.

12. Gerät nach Anspruch1, wobei der zylindrische Teil aus dem kugelförmigen Teil im Wesentlichen senkrecht zu der Längsachse des Schafts herausragt.

13. Gerät nach Anspruch 1, wobei der kugelförmige Teil mindestens einen Teil jeweils von der ersten Elektrode, der zweiten Elektrode und des Isolierstücks einschließt, und wobei der zylindrische Teil mindestens einen Teil jeweils von der ersten Elektrode, der zweiten Elektrode und des Isolierstücks einschließt.

14. Gerät nach Anspruch 1, wobei die erste Elektrode und die zweite Elektrode an jeweiligen Schnittstellen mit dem Isolierstück abgerundete Kanten haben, und wobei der zylindrische Teil an einem Endstück davon eine abgerundete Kante hat.

15. Gerät nach Anspruch 7, wobei das Isolierstück im Wesentlichen den gesamten zylindrischen Teil bildet, und der kugelförmige Teil schließt mindestens einen Teil jeweils von der ersten Elektrode und der zweiten Elektrode ein.

## Revendications

1. Dispositif électrochirurgical bipolaire (10) comprenant :
une tige (170) ayant une extrémité proximale et une extrémité distale ; et
un embout à électrode (100) couplé à l'extrémité distale de la tige, de sorte qu'au moins une portion de l'embout à électrode s'étend en direction distale au-delà de l'extrémité distale de la tige,
dans lequel la portion de l'embout à électrode (100) comprend une première électrode (110), une seconde électrode (120), et un isolateur (130) disposé entre la première électrode et la seconde électrode,
dans lequel la première électrode (110) est configurée pour être une électrode active et la seconde électrode (120) est configurée pour être une électrode de retour ; et
dans lequel au moins la portion de l'embout à électrode inclut une portion de forme sensiblement conique (104) qui inclut au moins une portion de chacune de la première et de la seconde électrode, ou
la portion de l'embout à électrode inclut une portion sphérique (102, 210) et une portion cylindrique (106, 220) qui se projette depuis la portion sphérique (102, 210) sous un angle différent de zéro par rapport à un axe longitudinal de la tige.

2. Dispositif selon la revendication 1, dans lequel l'extrémité distale de la tige inclut une ouverture de sortie de fluide (160) en communication fluidique avec une source de fluide, l'ouverture de sortie de fluide étant configurée pour amener du fluide depuis la source de fluide jusque sur une zone à proximité de la première électrode et de la seconde électrode.

3. Dispositif selon la revendication 2, dans lequel le fluide est électriquement conducteur et/ou inclut des sels.

4. Dispositif selon la revendication 1, dans lequel la première électrode et la seconde électrode ont des bords arrondis à des interfaces respectives avec l'isolateur.

5. Dispositif selon la revendication 1, dans lequel isolateur s'étend parallèlement à un axe longitudinal de l'embout à électrode, et dans lequel la première électrode et la seconde électrode sont disposées sur des côtés latéralement opposés de l'isolateur ; dans lequel isolateur est en option centré par rapport à l'axe longitudinal de l'embout à électrode, et/ou l'isolateur a une épaisseur uniforme.

6. Dispositif selon la revendication 1, dans lequel l'embout à électrode (100) comprend une portion sphérique (102) à une extrémité distale de lui-même, dans lequel une extrémité distale de la portion à forme sensiblement conique (104) est couplée à une extrémité proximale de la portion sphérique (102), et dans lequel une extrémité distale d'une portion cylindrique (106) est couplée à une extrémité proximale de la portion à forme sensiblement conique (104).

7. Dispositif selon la revendication 1, dans lequel isolateur (140) s'étend transversalement par rapport à un axe longitudinal de l'embout à électrode (100), et dans lequel la première électrode et la seconde électrode sont disposées sur des côtés longitudinalement opposés de l'isolateur ; dans lequel l'isolateur s'étend en option sous un angle oblique par rapport à l'axe longitudinal de l'embout à électrode.

8. Dispositif selon la revendication 1, dans lequel l'une au moins de la première électrode et de la seconde électrode est formée d'une encre conductrice disposée sur un substrat.

9. Dispositif selon la revendication 2, dans lequel l'embout à électrode comprend une portion sphérique à son extrémité distale, dans lequel une extrémité distale de la portion à forme sensiblement conique est couplée à une extrémité proximale de la portion sphérique, et dans lequel une extrémité distale d'une portion cylindrique est couplée à une extrémité proximale de la portion à forme sensiblement conique ; dans lequel la portion à forme sensiblement conique inclut en option sensiblement la totalité de la première électrode, et dans lequel la portion cylindrique inclut sensiblement la totalité de la seconde électrode.

10. Dispositif selon la revendication 2, dans lequel l'isolateur s'étend parallèlement à un axe longitudinal de l'embout à électrode, et dans lequel la première électrode et la seconde électrode sont disposées sur des côtés latéralement opposés de l'isolateur.

11. Dispositif selon la revendication 2, dans lequel le fluide est électriquement conducteur, et la première électrode et la seconde électrode sont configurées pour être excitées par une énergie à radiofréquences.

12. Dispositif selon la revendication 1, dans lequel la portion cylindrique se projette depuis la portion sphérique sensiblement perpendiculairement à l'axe longitudinal de la tige.

13. Dispositif selon la revendication 1, dans lequel la portion sphérique inclut au moins une portion de chacune/chacun de la première électrode, de la seconde électrode, et de l'isolateur, et dans lequel la portion cylindrique inclut au moins une portion de chacune/chacun de la première électrode, de la seconde électrode, et de l'isolateur.

14. Dispositif selon la revendication 1, dans lequel la première électrode et la seconde électrode ont des bords arrondis à des interfaces respectives avec isolateur, et dans lequel la portion cylindrique a un bord arrondi à une portion terminale d'elle-même.

15. Dispositif selon la revendication 7, dans lequel isolateur forme sensiblement la totalité de la portion cylindrique, et la portion sphérique inclut au moins une portion de chacune de la première électrode et de la seconde électrode.
